Europäisches Patentamt

European Patent Office

Office européen des brevets

(19)

(11) Veröffentlichungsnummer: **0 133 284**
**A2**

# (12) EUROPÄISCHE PATENTANMELDUNG

(21) Anmeldenummer: **84108768.7**

(51) Int. Cl.⁴: **C 07 K 3/20**, C 12 P 21/02

(22) Anmeldetag: **25.07.84**

(30) Priorität: **29.07.83 DE 3327710**

(43) Veröffentlichungstag der Anmeldung: **20.02.85**
**Patentblatt 85/8**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt am Main 80 (DE)**

(72) Erfinder: **Obermeier, Rainer, Dr., Langenhainer Weg 14, D-6234 Hattersheim am Main (DE)**
Erfinder: **Leineweber, Michael, Dr., Heimchenweg 74, d-6230 Frankfurt am Main 80 (DE)**
Erfinder: **Rolly, Heinrich, Dr., Am Tulpengarten 18, D-6233 Kelkheim (Taunus) (DE)**

(54) **Verfahren zur Isolierung und Reinigung von Interferon.**

(57) Die Erfindung betrifft ein Verfahren zur chromatographischen Abtrennung und Reinigung von Interferon, durch Chromatographie des zu trennenden bzw. zu reinigenden Materials mit einem organischen Laufmittelgemisch an Kieselgel.

EP 0 133 284 A2

HOECHST AKTIENGESELLSCHAFT   HOE 83/F 150   Dr.WS/mk   0133284

Verfahren zur Isolierung und Reinigung von Interferon

Bereits 1956 endeckten Isaacs und Lindemann Interferon als einen indirekten Hemmstoff der intracellulären Virusvermehrung. Inzwischen wurde eine große Anzahl verschiedener Interferontypen natürlicher Herkunft identifiziert, die vor allem durch die unterschiedlichen Herkunftszellen charakterisiert werden:

$\alpha$-Interferone aus Leukozyten
ß-Interferone aus Fibroblasten
$\gamma$-Interferone aus Lymphozyten.

Die durch entsprechende Stimuli in den jeweiligen Zellen induzierten Interferone gehören chemisch zur Gruppe der Glykoproteine, $\alpha$-und  ß-Interferone sind säurestabil (pH 2) und entfalten ihre volle antivirale Wirkung von $10^9$ IE/mg speziesspezifisch. Die Primärstruktur einer Reihe von $\alpha$-, und ß-Interferonen, die aus 165 bzw. 166 Aminosäuren aufgebaut sind, ist aufgeklärt.

Jüngste Arbeiten auf dem Gebiet der Gentechnologie, bei denen Interferon-Gene in E.coli zur Expression gebracht wurden, haben gezeigt, daß die in den natürlichen Interferonen nachgewiesenen Glycosyl- bzw. Polysaccharid-Seitenketten keinen Einfluß auf die biologische Aktivität der Proteine zu haben scheinen. Darüberhinaus kann mit Antikörpern, die mit dem natürlichen Glykoprotein erzeugt wurden, eine vergleichbare Bindung an nicht glykosilierte Interferone aus E.coli gemessen werden, was auf den überwiegenden Einfluß der Proteinstruktur des Interferons als Antigendeterminante hindeutet.

Wegen ihrer therapeutischen Potenz werden die Interferone im
großtechnischen Maßstab entweder durch Ernten von Kulturmedien entsprechender Zellkulturen, oder neuerdings durch
Fermentation von Hefen und Bakterien (z.B. E.coli), in die
auf gentechnologischem Wege ein Interferon-codierender
DNA-Vektor cloniert wurde, produziert.

Zur Isolierung und Reinigung der gewonnenen Rohinterferonpräparate (durchschnittliche Aktivität $10^3$-$10^4$ IE/mg
Protein) werden im allgemeinen Verfahren der Affinitäts- und
Adsorptionschromatographie verwendet. Diese nützen die
spezielle Fähigkeit des Interferons an immobilisierte
hydrophobe Liganden, Metallionen, Thiole, organische Quecksilberverbindungen, Polynukleotide, Controlled Pore Glass,
so wie an Antikörper mit hoher Spezifität zu binden. Trotz
der Vielfalt von Reinigungsmethoden bleiben Ausbeuten und
Reinheit der damit erhaltenen Interferone jedoch unbefriedigend, wie die Unsicherheit über die Ursache der Nebenwirkungen bei der klinischen Prüfung der Interferone am Menschen
zeigt. Neue optimale Isolierungs- und Reinigungsverfahren
sind deshalb notwendig.

Aus Prep. Biochem. 5 [ 1975 ] 397 - 412 ist ein Verfahren zur
Reinigung einiger Peptide und Proteine durch Hochdruck-
Flüssigkeits-Chromatographie mit Methanol-Wasser-Ameisensäure-
Gemischen an handelsüblichen Kieselgel-Fertigsäulen bekannt.
Von den getesteten Sorbentien erwiesen sich Kieselgele mit
einer durchschnittlichen Partikelgröße größer als 10 /um
als unbrauchbar zur Trennung von Peptiden oder Proteinen.

In der EP-A-82 359 wird ein chromatographisches Reinigungsverfahren für Insulin bzw. Insulinderivate an Kieselgel
beschrieben. Bei diesem Verfahren werden peptidische Verunreinigungen und höhermolekulare Peptide (wie Enzyme) so
stark an Kieselgel adsorbiert, daß das Sorbens nach durchgeführter Trennung nicht mehr regeneriert sondern verworfen
wird.

Weiterhin ist aus der EP-A-71647 die spezifische Anreicherung von Interferonen durch Adsorption an Silikaten wie Bentonit bekannt.

Es war daher sehr überraschend, daß Interferon (Molekulargewicht 18000-22000) mit geeigneten organischen Lösungsmittelgemischen ein so gutes Laufverhalten an handelsüblichen Kieselgelen für die Säulenchromatographie zeigt, daß die chromatographische Auftrennung und Reinigung interferonhaltiger Rohgemische an Säulen gepackt mit solchen Kieselgelen erfolgreich durchführbar ist.

Die Erfindung betrifft daher ein Verfahren zur chromatographischen Abtrennung und Reinigung von Interferon, dadurch gekennzeichnet, daß das zu trennende bzw. zu reinigende Material mit einem organischen Laufmittelgemisch an Kieselgel chromatographiert wird.

Als Laufmittel können alle die Lösungsmittelgemische verwendet werden, in denen Interferon einerseits und die abzutrennende Verunreinigungen andererseits auf käuflichen Kieselgelplatten zur Dünnschichtchromatographie unterschiedliche $R_f$-Werte aufweisen.

Als geeignet hat sich beispielsweise ein Laufmittelgemisch erwiesen, das zusammengesetzt ist aus

a) einem niederen aliphatischen Halogenkohlenwasserstoff, vorzugsweise einem halogenierten ($C_1$ bis $C_3$)-Alkan, wie Chloroform, Methylenchlorid oder 1,1,1-Trichlorethan,
b) Methanol und/oder Ethanol,
c) gegebenenfalls Ethylenglykolmonomethylether (Methylglykol) und/oder Ethylenglykolmonoethylether (Ethylglykol),
d) Wasser
e) einer niederen aliphatischen Carbonsäure, vorzugsweise mit bis zu 3 C-Atomen, wie Essigsäure oder Ameisensäure und

f) Ammoniak und/oder einem bevorzugt leichtflüchtigen organischen Amin, vorzugsweise tertiären aliphatischen Amin mit bis zu 12 C-Atomen oder tertiären heterocyclischen Amin mit bis zu 10 C-Atomen, wie Triethylamin, N,N-Diethyl-N-allylamin, N,N-Diisopropyl-N-ethylamin oder N-Ethylmorpholin.

Unter tertiären aliphatischen Aminen werden insbesondere Alkyl- und Alkenylamine, unter tertiären heterocyclischen Aminen insbesondere N-Alkylderivate von Pyrrol, Pyrrolin, Pyrrolidin, Piperidin und Morpholin verstanden.

Bevorzugt ist ein Laufmittelgemisch aus

a) 300 bis 800 Volumenteilen eines niederen aliphatischen Halogenkohlenwasserstoffs,
b) 400 bis 700 Volumenteilen Methanol und/oder Ethanol,
c) 100 bis 300 Volumenteilen Wasser,
d) 0 bis 150 Volumenteilen Methylglykol und/oder Ethylglykol,
e) 1 bis 25 Volumenteile einer niederen aliphatischen Carbonsäure und
f) 1 bis 5 Gewichtsteile Ammoniak, der ganz oder teilweise durch die äquimolare Menge eines leichtflüchtigen organischen Amins ersetzt sein kann.

Ammoniak bzw. ein Amin kann auch als Salz der unter e) genannten Carbonsäure eingesetzt werden.

Besonders bevorzugt ist ein Laufmittelgemisch aus Chloroform, und/oder Methylenchlorid, Methanol, Wasser, Methylglykol, Essigsäure, Triethylamin und Ammoniumacetat, insbesondere aus

a) 300 bis 800 Volumenteilen Chloroform und/oder Methylenchlorid,

- 5 -                    0133284

b) 400 bis 700 Volumenteilen Methanol,
c) 100 bis 300 Volumenteilen Wasser,
d)   0 bis 150 Volumenteilen Ethylenglykolmonomethylether,
e)   1 bis  15 Volumenteilen Essigsäure,
f)   3 bis   8 Volumenteilen Triethylamin und
     1 bis  10 Gewichtsteilen Ammoniumacetat.

Die Überlegenheit des erfindungsgemäßen Verfahrens basiert auf der spezifischen Verhinderung der Adsorption von Interferon an Kieselgel durch Verwendung von entsprechenden organischen Laufmittelsystemen, in denen Interferon überraschenderweise löslich bleibt. Das erfindungsgemäße Verfahren ist daher hervorragend zur Trennung von Gemischen aus Interferon und den bei seiner Gewinnung aus Zellkulturen, Hefe- bzw. Bakterienaufschlüssen und Fermenterbrühen anfallenden Verunreinigungen geeignet.

Interferonhaltiges Rohmaterial sind vor allem gentechnologisch veränderte Bakterien- oder Hefekulturen und Kulturüberstände von induzierten Säugetierzellen.

Besonders vorteilhaft ist das Verfahren zur Abtrennung von Interferon aus Fermentation von gentechnologisch modifiziertem E.coli-Stämmen anzuwenden.

Als Säulenfüllung kommt jedes käufliche Kieselgel in Betracht, wie beispielsweise das Handelprodukt $SiO_2$ 60, Merck, Darmstadt mit einem Korngrößebereich 0,04 - 0,20 mm oder Grace Silica Gels for Chromatographie mit einem Korngrößebereich 0,035 - 0,10 mm.

Da die Säulenfüllungen nach mehreren aufeinanderfolgenden Läufen zum Teil irreversibel mit Fremdprotein beladen sind, kann das Kieselgel verworfen werden. Die Kosten spielen gegenüber dem verarbeiteten Interferon keine Rolle. Als zusätzlicher Vorteil ergibt sich dabei, daß die Trenneigenschaften der Kieselgelsäule konstant gut bleiben. Ferner lassen sich Proteinkontaminationen, wie sie bei häufiger Regenerierung teurer Säulenfüllmaterialien unvermeidbar sind, ausschließen.

Ausführungsbeispiele:

Beispiel 1

Lysat einer 10 l Fermentation eines E.coli-Stammes der nach gentechnologischer Manipulation Interferon exprimiert, wird zentrifugiert und der klare Überstand gefriergetrocknet.

Der Proteingehalt dieses Rohprodukts wird nach der Lowry-Methode zu 50 % bestimmt. Der mit Hilfe der Reduktion des cytopathischen Effekts (Lin et al. J. Gen. Virol. 39 125-130, (1978) gemessene Interferongehalt beträgt $3,5 \cdot 10^9$ IF E/g Protein.

2 g des Rohmaterials werden in 5 ml des Laufmittelsystems (600 ml Chloroform, 570 ml Methanol, 200 ml Wasser, 9 g Ammoniumacetat, 5,5 ml Triethylamin, 2 ml Essigsäure) im Ultraschallbad suspendiert. Die trübe Lösung wird zentrifugiert und der erhaltene klare Überstand gesammelt. Der Rückstand wird erneut mit 2 ml Laufmittel unter Zusatz von 0,5 ml Essigsäure extrahiert und zentrifugiert. Die vereinigten klaren Überstände werden auf eine Säule (5x80 cm), die mit im Laufmittel aufgeschlämmten Kieselgel-60 der Firma Merck gefüllt wurde, aufgetragen. Der unlösliche Niederschlag nach Zentrifugation wird im Vakuum getrocknet: Auswaage 1,03 g. Die beladene Kieselgelsäule wird durch Aufpumpen von Laufmittel unter leichtem Überdruck entwickelt. Die Kontrolle des Eluates erfolgt, wie üblich, im Durchfluß-UV-Spektrometer bei 254 nm. Wenige Fraktionen nach dem Leervolumen der Säule erscheint ein Peak der die gesamte Interferonaktivität enthält. Weitere Produkte eluieren mit verzögerter Retentionszeit. Sie enthalten keine Interferonaktivität und werden nicht identifiziert.

Man verdünnt das gesammelte interferonhaltige Eluat mit bidestilliertem, pyrogenfreiem Wasser auf etwa das doppelte Volumen und dampft anschließend im Vakuum bei Raumtemperatur auf ungefähr die Hälfte dieses Gesamtvolumens ein. Danach sind die organischen Lösungsmittel soweit entfernt, daß die verbleibende wäßrige Lösung problemlos gefriergetrocknet werden kann. Ausbeute nach Gefriertrocknung 10 mg (Proteingehalt: 60 %); Interferonaktivität $2,0 \cdot 10^8$ IF E/mg Protein. Ohne Berücksichtigung der Testgenauigkeit beträgt also die Ausbeute an Interferon ca. 50-60 % der auf die Säule aufgetragenen Ausgangsmenge. Nach wiederholter Reinigungsprozedur zeigt das Material in der SDS-Elektrophorese eine weitgehend einheitliche Bande bei ca. 18 K Dalton. Die Aminosäureanalyse entspricht den theoretischen Werten. Die biologische Aktivität beträgt $8,5 \cdot 10^8$ IF E/mg.

Beispiel 2

Gefriergetrocknetes interferonhaltiges Rohmaterial aus Sendai-Virus induzierten Leukocytenkulturen wurde als Ausgangsmaterial für die Interferon-Reinigung verwendet. Das Material enthielt 62 % Protein und $2 \cdot 10^5$ IF E/mg Protein.

1,6 g der Rohsubstanz wurden wie in Beispiel 1 chromatographiert und die interferonhaltigen Fraktionen aufgearbeitet. Ausbeute 2 mg Substanz; 55 % Proteingehalt; $10^8$ IF E/mg.

### Beispiel 3

2 g interferonhaltiges Rohmaterial aus E.coli werden wie in Beispiel 1 chromatographiert und aufgearbeitet. Als Laufmittel wird ein Gemisch verwendet, bei dem das Chloroformvolumen in Beispiel 1 durch das gleiche Volumen Methylenchlorid ersetzt wurde. Ausbeute: 15 mg Interferon; 40 % Proteingehalt; $10^8$ IF E/mg Protein.

### Beispiel 4

2 g interferonhaltiges Rohmaterial aus E.coli werden wie in Beispiel 1 chromatographiert und dabei wird die Menge an Ammoniumacetat von 9 g wie in Beispiel 1 auf 2g reduziert. Ausbeute: 6 mg Interferon; 70 % Proteingehalt, $5 \cdot 10^8$ IF E/mg Protein.

Patentansprüche:

1. Verfahren zur chromatographischen Abtrennung und Reinigung von Interferon, dadurch gekennzeichnet, daß das zu trennende bzw. zu reinigende Material mit einem organischen Laufmittelgemisch an Kieselgel chromatographiert wird.

2. Verfahren gemäß Anspruch 1, dadurch gekennzeichnet, daß man an einer mit Kieselgel gefüllten Säule chromatographiert.

3. Verfahren gemäß Anspruch 1 oder 2, dadurch gekennzeichnet, daß die Chromatographie mit einem Laufmittelgemisch erfolgt, das zusammengesetzt ist aus

a) einem niederen aliphatischen Halogenkohlenwasserstoff,
b) Methanol und/oder Ethanol,
c) ggfs. Ethylenglykolmonomethylether und/oder Ethylenglykolmonoethylether,
d) Wasser,
e) einer niederen aliphatischen Carbonsäure und
f) Ammoniak und/oder einem organischen Amin.

4. Verfahren gemäß einem der Ansprüche 1 bis 3, dadurch gekennzeichnet, daß die Chromatographie mit einem Laufmittelgemisch erfolgt, das zusammengesetzt ist aus

a) 300 bis 800 Volumenteilen eines niederen aliphatischen Halogenkohlenwasserstoffs,
b) 400 bis 700 Volumenteilen Methanol und/oder Ethanol,
c) 100 bis 300 Volumenteilen Wasser,
d) 0 bis 150 Volumenteilen Ethylenglykolmonomethylether und/oder -ethylether
e) 1 bis 25 Volumenteile einer niederen aliphatischen Carbonsäure und
f) 1 bis 5 Gewichtsteile Ammoniak, der ganz oder teilweise durch die äquimolare Menge eines leichtflüchtigen organischen Amins ersetzt sein kann.

5. Verfahren gemäß einem der Ansprüche 1 bis 3, dadurch gekennzeichnet, daß die Chromatographie mit einem Laufmittelgemisch erfolgt, das zusammengesetzt ist aus Chloroform und/oder Methylenchlorid, Methanol, Wasser, gegebenenfalls Ethylenglykolmonomethylether, Essigsäure, Triethylamin und Ammoniumacetat.

6. Verfahren gemäß einem der Ansprüche 1 bis 5, dadurch gekennzeichnet, daß die Chromatographie mit einem Laufmittelgemisch erfolgt, das zusammengesetzt ist aus

   a) 300 bis 800 Volumenteilen Chloroform und/oder Methylenchlorid,
   b) 400 bis 700 Volumenteilen Methanol,
   c) 100 bis 300 Volumenteilen Wasser,
   d)   0 bis 150 Volumenteilen Ethylenglykolmonomethylether,
   e)   1 bis  15 Volumenteilen Essigsäure,
   f)   3 bis   8 Volumenteilen Triethylamin und
        1 bis  10 Gewichtsteilen Ammoniumacetat.

7. Verfahren gemäß einem der Ansprüche 1 bis 6, dadurch gekennzeichnet, daß an Kieselgel mit einer Korngröße von 0,03 bis 0,2 mm chromatographiert wird.

8. Verfahren gemäß einem der Ansprüche 1 bis 7, dadurch gekennzeichnet, daß Interferon aus gentechnologisch veränderten Bakterien- bzw. Hefekulturen abgetrennt und gereinigt wird.

9. Verfahren gemäß einem der Ansprüche 1 bis 7, dadurch gekennzeichnet, daß Interferon aus Kulturüberständen von induzierten Säugetierzellen abgetrennt und gereinigt wird.